# EUROPEAN PATENT APPLICATION

(11) **EP 2 383 578 A1**
(43) Date of publication of application: **02.11.2011**
(21) Application number: 10161559.9
(22) Date of filing: 30.04.2010
(51) Int. Cl.: G01N 33/574

(54) **Tumor marker and methods of use thereof**

(71) Applicant: Externautics S.p.A., 53100 Siena (IT)
(72) Inventor: Grifantini, Renata, 53100 Siena (IT); Pileri, Piero, 53100 Siena (IT); Campagnoli, Susanna, 53100 Siena (IT); Parri, Matteo, 53100 Siena (IT); Grandi, Alberto, 53100 Siena (IT); Pierleoni, Andrea, 53100 Siena (IT)
(74) Representative: Banfi, Paolo

(57) **Abstract**

Newly identified proteins as markers for the detection of breast, colon, lung and ovary tumors, or as therapeutic targets for their treatment, affinity ligands capable of selectively interacting with the newly identified markers and methods for tumor diagnosis and therapy using such ligands.

SEQUENCE LISTING

<110>  EXTERNAUTICS S.p.A.

<120>  Tumor marker and methods of use thereof

<130>  2342EUR

<160>  8

<170>  PatentIn version 3.3

<210>  1
<211>  357
<212>  PRT
<213>  Homo sapiens

<400>  1

Met Glu Asn Lys Ala Met Tyr Leu His Thr Val Ser Asp Cys Asp Thr
1               5                   10                  15

Ser Ser Ile Cys Glu Asp Ser Phe Asp Gly Arg Ser Leu Ser Lys Leu
            20                  25                  30

Asn Leu Cys Glu Asp Val Ser Arg Pro Arg Ser Ser Pro Asp Asp Leu
        35                  40                  45

Lys Ala Leu Thr Arg Asn Val Asn Arg Leu Asn Glu Ser Phe Arg Asp
    50                  55                  60

Leu Gln Leu Arg Leu Leu Gln Ala Pro Leu Gln Ala Asp Leu Thr Glu
65                  70                  75                  80

Gln Val Trp Lys Val Gln Asp Ala Leu Gln Asn Gln Ser Asp Ser Leu
                85                  90                  95

Leu Ala Leu Ala Gly Ala Val Gln Arg Leu Glu Gly Ala Leu Trp Gly
            100                 105                 110

Leu Gln Ala Gln Ala Val Gln Thr Glu Gln Ala Val Ala Leu Leu Arg
        115                 120                 125

Asp Arg Thr Gly Gln Gln Ser Asp Thr Ala Gln Leu Glu Leu Tyr Gln
    130                 135                 140

Leu Gln Val Glu Ser Asn Ser Ser Gln Leu Leu Leu Arg Arg His Ala
145                 150                 155                 160

Gly Leu Leu Asp Gly Leu Ala Arg Arg Val Gly Ile Leu Gly Glu Glu
                165                 170                 175

Leu Ala Asp Val Gly Gly Val Leu Arg Gly Leu Asn His Ser Leu Ser
            180                 185                 190

Tyr Asp Val Ala Leu His Arg Thr Arg Leu Gln Asp Leu Arg Val Leu
        195                 200                 205

Val Ser Asn Ala Ser Glu Asp Thr Arg Arg Leu Arg Leu Ala His Val
    210                 215                 220

Gly Met Glu Leu Gln Leu Lys Gln Glu Leu Ala Met Leu Asn Ala Val
225                 230                 235                 240

Thr Glu Asp Leu Arg Leu Lys Asp Trp Glu His Ser Ile Ala Leu Arg
                245                 250                 255

Asn Ile Ser Leu Ala Lys Gly Pro Pro Gly Pro Lys Gly Asp Gln Gly
            260                 265                 270

Asp Glu Gly Lys Glu Gly Arg Pro Gly Ile Pro Gly Leu Pro Gly Leu
        275                 280                 285

Arg Gly Leu Pro Gly Glu Arg Gly Thr Pro Gly Leu Pro Gly Pro Lys
    290                 295                 300

Gly Asp Asp Gly Lys Leu Gly Ala Thr Gly Pro Met Gly Met Arg Gly
305                 310                 315                 320

Phe Lys Gly Asp Arg Gly Pro Lys Gly Glu Lys Gly Glu Lys Gly Asp
                325                 330                 335

Arg Ala Gly Asp Ala Ser Lys Asp Ile Leu Leu Gly Pro Trp Asp Met
            340                 345                 350

Val Leu Ala Gln Gly
        355

<210>  2
<211>  270
<212>  PRT
<213>  Homo sapiens

<400>  2

Met Glu Asn Lys Ala Met Tyr Leu His Thr Val Ser Asp Cys Asp Thr
1               5                   10                  15

Ser Ser Ile Cys Glu Asp Ser Phe Asp Gly Arg Ser Leu Ser Lys Leu
            20                  25                  30

Asn Leu Cys Glu Asp Gly Pro Cys His Lys Arg Arg Ala Ser Ile Cys
        35                  40                  45

Cys Thr Gln Leu Gly Ser Leu Ser Ala Leu Lys His Ala Val Leu Gly
    50                  55                  60

Leu Tyr Leu Leu Val Phe Leu Ile Leu Val Gly Ile Phe Ile Leu Ala
65                  70                  75                  80

Gly Pro Pro Gly Pro Lys Gly Asp Gln Gly Asp Glu Gly Lys Glu Gly
                85                  90                  95

Arg Pro Gly Ile Pro Gly Leu Pro Gly Leu Arg Gly Leu Pro Gly Glu
            100                 105                 110

Arg Gly Thr Pro Gly Leu Pro Gly Pro Lys Gly Asp Asp Gly Lys Leu
        115                 120                 125

Gly Ala Thr Gly Pro Met Gly Met Arg Gly Phe Lys Gly Asp Arg Gly
    130                 135                 140

Pro Lys Gly Glu Lys Gly Glu Lys Gly Asp Arg Ala Gly Asp Ala Ser
145                 150                 155                 160

Gly Val Glu Ala Pro Met Met Ile Arg Leu Val Asn Gly Ser Gly Pro
                165                 170                 175

His Glu Gly Arg Val Glu Val Tyr His Asp Arg Arg Trp Gly Thr Val
            180                 185                 190

Cys Asp Asp Gly Trp Asp Lys Lys Asp Gly Asp Val Val Cys Arg Met
        195                 200                 205

Leu Gly Phe Arg Gly Val Glu Glu Val Tyr Arg Thr Ala Arg Phe Gly
    210                 215                 220

Gln Gly Thr Gly Arg Ile Trp Met Asp Asp Val Ala Cys Lys Gly Thr
225                 230                 235                 240

Glu Glu Thr Ile Phe Arg Cys Ser Phe Ser Lys Trp Gly Val Thr Asn
                245                 250                 255

Cys Gly His Ala Glu Asp Ala Ser Val Thr Cys Asn Arg His
            260                 265                 270

<210>  3
<211>  495

<212>  PRT
<213>  Homo sapiens

<400>  3

Met Glu Asn Lys Ala Met Tyr Leu His Thr Val Ser Asp Cys Asp Thr
1               5                   10                  15

Ser Ser Ile Cys Glu Asp Ser Phe Asp Gly Arg Ser Leu Ser Lys Leu
            20                  25                  30

Asn Leu Cys Glu Asp Gly Pro Cys His Lys Arg Arg Ala Ser Ile Cys
        35                  40                  45

Cys Thr Gln Leu Gly Ser Leu Ser Ala Leu Lys His Ala Val Leu Gly
    50                  55                  60

Leu Tyr Leu Leu Val Phe Leu Ile Leu Val Gly Ile Phe Ile Leu Ala
65                  70                  75                  80

Val Ser Arg Pro Arg Ser Ser Pro Asp Asp Leu Lys Ala Leu Thr Arg
                85                  90                  95

Asn Val Asn Arg Leu Asn Glu Ser Phe Arg Asp Leu Gln Leu Arg Leu
            100                 105                 110

Leu Gln Ala Pro Leu Gln Ala Asp Leu Thr Glu Gln Val Trp Lys Val
        115                 120                 125

Gln Asp Ala Leu Gln Asn Gln Ser Asp Ser Leu Leu Ala Leu Ala Gly
    130                 135                 140

Ala Val Gln Arg Leu Glu Gly Ala Leu Trp Gly Leu Gln Ala Gln Ala
145                 150                 155                 160

Val Gln Thr Glu Gln Ala Val Ala Leu Leu Arg Asp Arg Thr Gly Gln
                165                 170                 175

Gln Ser Asp Thr Ala Gln Leu Glu Leu Tyr Gln Leu Gln Val Glu Ser
            180                 185                 190

Asn Ser Ser Gln Leu Leu Leu Arg Arg His Ala Gly Leu Leu Asp Gly
        195                 200                 205

Leu Ala Arg Arg Val Gly Ile Leu Gly Glu Glu Leu Ala Asp Val Gly
    210                 215                 220

Gly Val Leu Arg Gly Leu Asn His Ser Leu Ser Tyr Asp Val Ala Leu
225                 230                 235                 240

His Arg Thr Arg Leu Gln Asp Leu Arg Val Leu Val Ser Asn Ala Ser
                245                 250                 255

Glu Asp Thr Arg Arg Leu Arg Leu Ala His Val Gly Met Glu Leu Gln
            260                 265                 270

Leu Lys Gln Glu Leu Ala Met Leu Asn Ala Val Thr Glu Asp Leu Arg
        275                 280                 285

Leu Lys Asp Trp Glu His Ser Ile Ala Leu Arg Asn Ile Ser Leu Ala
    290                 295                 300

Lys Gly Pro Pro Gly Pro Lys Gly Asp Gln Gly Asp Glu Gly Lys Glu
305                 310                 315                 320

Gly Arg Pro Gly Ile Pro Gly Leu Pro Gly Leu Arg Gly Leu Pro Gly
                325                 330                 335

Glu Arg Gly Thr Pro Gly Leu Pro Gly Pro Lys Gly Asp Asp Gly Lys
            340                 345                 350

Leu Gly Ala Thr Gly Pro Met Gly Met Arg Gly Phe Lys Gly Asp Arg
        355                 360                 365

Gly Pro Lys Gly Glu Lys Gly Glu Lys Gly Asp Arg Ala Gly Asp Ala
    370                 375                 380

Ser Gly Val Glu Ala Pro Met Met Ile Arg Leu Val Asn Gly Ser Gly
385                 390                 395                 400

Pro His Glu Gly Arg Val Glu Val Tyr His Asp Arg Arg Trp Gly Thr
                405                 410                 415

Val Cys Asp Asp Gly Trp Asp Lys Lys Asp Gly Asp Val Val Cys Arg
            420                 425                 430

Met Leu Gly Phe Arg Gly Val Glu Glu Val Tyr Arg Thr Ala Arg Phe

        435                 440                 445

Gly Gln Gly Thr Gly Arg Ile Trp Met Asp Asp Val Ala Cys Lys Gly
    450                 455                 460

Thr Glu Glu Thr Ile Phe Arg Cys Ser Phe Ser Lys Trp Gly Val Thr
465                 470                 475                 480

Asn Cys Gly His Ala Glu Asp Ala Ser Val Thr Cys Asn Arg His
                485                 490                 495

<210>  4
<211>  400
<212>  PRT
<213>  Homo sapiens

<400>  4

Met Glu Asn Lys Ala Met Tyr Leu His Thr Val Ser Asp Cys Asp Thr
1               5                   10                  15

Ser Ser Ile Cys Glu Asp Ser Phe Asp Gly Arg Ser Leu Ser Lys Leu
            20                  25                  30

Asn Leu Cys Glu Asp Gly Pro Cys His Lys Arg Arg Ala Ser Ile Cys
        35                  40                  45

Cys Thr Gln Leu Gly Ser Leu Ser Ala Leu Lys His Ala Val Leu Gly
    50                  55                  60

Leu Tyr Leu Leu Val Phe Leu Ile Leu Val Gly Ile Phe Ile Leu Ala
65                  70                  75                  80

Val Ser Arg Pro Arg Ser Ser Pro Asp Asp Leu Lys Ala Leu Thr Arg
                85                  90                  95

Asn Val Asn Arg Leu Asn Glu Ser Phe Arg Asp Leu Gln Leu Arg Leu
            100                 105                 110

Leu Gln Ala Pro Leu Gln Ala Asp Leu Thr Glu Gln Val Trp Lys Val
        115                 120                 125

Gln Asp Ala Leu Gln Asn Gln Ser Asp Ser Leu Leu Ala Leu Ala Gly
    130                 135                 140

Ala Val Gln Arg Leu Glu Gly Ala Leu Trp Gly Leu Gln Ala Gln Ala
145                 150                 155                 160

Val Gln Thr Glu Gln Ala Val Ala Leu Leu Arg Asp Arg Thr Gly Gln
                165                 170                 175

Gln Ser Asp Thr Ala Gln Leu Glu Leu Tyr Gln Leu Gln Val Glu Ser
            180                 185                 190

Asn Ser Ser Gln Leu Leu Leu Arg Arg His Ala Gly Leu Leu Asp Gly
        195                 200                 205

Leu Ala Arg Arg Val Gly Ile Leu Gly Glu Glu Leu Ala Asp Val Gly
    210                 215                 220

Gly Val Leu Arg Gly Leu Asn His Ser Leu Ser Tyr Asp Val Ala Leu
225                 230                 235                 240

His Arg Thr Arg Leu Gln Asp Leu Arg Val Leu Val Ser Asn Ala Ser
                245                 250                 255

Glu Asp Thr Arg Arg Leu Arg Leu Ala His Val Gly Met Glu Leu Gln
            260                 265                 270

Leu Lys Gln Glu Leu Ala Met Leu Asn Ala Val Thr Glu Asp Leu Arg
        275                 280                 285

Leu Lys Asp Trp Glu His Ser Ile Ala Leu Arg Asn Ile Ser Leu Ala
    290                 295                 300

Lys Gly Pro Pro Gly Pro Lys Gly Asp Gln Gly Asp Glu Gly Lys Glu
305                 310                 315                 320

Gly Arg Pro Gly Ile Pro Gly Leu Pro Gly Leu Arg Gly Leu Pro Gly
                325                 330                 335

Glu Arg Gly Thr Pro Gly Leu Pro Gly Pro Lys Gly Asp Asp Gly Lys
            340                 345                 350

Leu Gly Ala Thr Gly Pro Met Gly Met Arg Gly Phe Lys Gly Asp Arg
        355                 360                 365

Gly Pro Lys Gly Glu Lys Gly Glu Lys Gly Asp Arg Ala Gly Asp Ala
    370                 375                 380

Ser Lys Asp Ile Leu Leu Gly Pro Trp Asp Met Val Leu Ala Gln Gly
385                 390                 395                 400


<210>  5
<211>  1514
<212>  DNA
<213>  Homo sapiens

<400>  5
tttattttat acggactggc ggcgagagca gctgcagttc gcatctcagg cagtacctag      60

aggagctgcc ggtgcctcct cagaacatct cctgatcgct acccaggacc aggcaccaag     120

gacagggagt cccaggcgca caccccccat tctgggtccc ccaggcccag acccccactc     180

tgccacaggt tgcatcttga cctggtcctc ctgcagaagt ggcccctgtg gtcctgctct     240

gagactcgtc cctgggcgcc cctgcagccc ctttctatga ctccatctgg atttggctgg     300

ctgtggggac gcggtccgag gggcggcctg gctctcagcg tggtggcagc cagctctctg     360

gccaccatgg caaatgctga gatctgaggg gacaaggctc tacagcctca gccaggggca 420

ctcagctgtt gcagggtgtg atggagaaca aagctatgta cctacacacc gtcagcgact 480

gtgacaccag ctccatctgt gaggattcct ttgatggcag gagcctgtcc aagctgaacc 540

tgtgtgagga tgtgtccagg ccgcgcagct cccctgacga cctgaaggcc ctgactcgca 600

atgtgaaccg gctgaatgag agcttccggg acttgcagct gcggctgctg caggctccgc 660

tgcaagcgga cctgacggag caggtgtgga aggtgcagga cgcgctgcag aaccagtcag 720

actcgttgct ggcgctggcg ggcgcagtgc agcggctgga gggcgcgctg tgggggctgc 780

aggcgcaggc ggtgcagacc gagcaggcgg tggccctgct gcgggaccgc acgggccagc 840

agagcgacac ggcgcagctg gagctctacc agctgcaggt ggagagcaac agtagccagc 900

tgctgctgag gcgccacgcg ggcctgctgg acgggctggc gcgcagggtg ggcatcctgg 960

gcgaggagct ggccgacgtg ggcggcgtgc tgcgcggcct caaccacagc ctgtcctacg 1020

acgtggccct ccaccgcacg cggctgcagg acctgcgggt gctggtgagc aacgccagcg 1080

aggacacgcg ccgcctgcgc ctggcgcacg taggcatgga gctgcagctg aagcaggagc 1140

tggccatgct caacgcggtc accgaggacc tgcgcctcaa ggactgggag cactccatcg 1200

cactgcggaa catctccctc gcgaaagggc caccgggacc caaaggtgat cagggggatg 1260

aaggaaagga aggcaggcct ggcatccctg gattgcctgg acttcgaggt ctgcccgggg 1320

agagaggtac cccaggattg cccgggccca agggcgatga tgggaagctg ggggccacag 1380

gaccaatggg catgcgtggg ttcaaaggtg accgaggccc aaaaggagag aaaggagaga 1440

aaggagacag agctggggat gccagtaagg acattctgct ggggccgtgg gatatggtgt 1500

tggcacaggg ctag 1514

<210> 6
<211> 2951
<212> DNA
<213> Homo sapiens

<400> 6
tttattttat acggactggc ggcgagagca gctgcagttc gcatctcagg cagtacctag 60

aggagctgcc ggtgcctcct cagaacatct cctgatcgct acccaggacc aggcaccaag 120

gacagggagt cccaggcgca caccccccat tctgggtccc ccaggcccag acccccactc 180

tgccacaggt tgcatcttga cctggtcctc ctgcagaagt ggcccctgtg gtcctgctct 240

gagactcgtc cctgggcgcc cctgcagccc ctttctatga ctccatctgg atttggctgg 300

ctgtggggac gcggtccgag ggcggcctgg gctctcagcg tggtggcagc cagctctctg 360

gccaccatgg caaatgctga gatctgaggg gacaaggctc tacagcctca gccaggggca 420

ctcagctgtt gcagggtgtg atggagaaca aagctatgta cctacacacc gtcagcgact 480

gtgacaccag ctccatctgt gaggattcct ttgatggcag gagcctgtcc aagctgaacc 540

tgtgtgagga tggtccatgt cacaaacggc gggcaagcat ctgctgtacc cagctggggt 600

ccctgtcggc cctgaagcat gctgtcctgg ggctctacct gctggtcttc ctgattcttg 660

tgggcatctt catcttagca gggccaccgg gacccaaagg tgatcagggg gatgaaggaa 720

aggaaggcag gcctggcatc cctggattgc ctggacttcg aggtctgccc ggggagagag 780

gtaccccagg attgcccggg cccaagggcg atgatgggaa gctgggggcc acaggaccaa 840

tgggcatgcg tgggttcaaa ggtgaccgag gcccaaaagg agagaaagga gagaaaggag 900

acagagctgg ggatgccagt ggcgtggagg ccccgatgat gatccgcctg gtgaatggct 960

caggtccgca cgagggccgc gtggaagtgt accacgaccg gcgttggggc accgtgtgtg 1020

acgacggctg ggacaagaag gacggagacg tggtgtgccg catgctcggc ttccgcggtg 1080

tggaggaggt gtaccgcaca gctcgattcg ggcaaggcac tgggaggatc tggatggatg 1140

acgttgcctg caagggcaca gaggaaacca tcttccgctg cagcttctcc aaatgggggg 1200

tgacaaactg tggacatgcc gaagatgcca gcgtgacatg caacagacac tgaaagtggg 1260

cagagcccaa gttcggggtc ctgcacagag cacccttcct gcatccctgg ggtggggcac 1320

agctcggggc caccctgacc atgcctcgac cacaccccgt ccagcattct cagtcctcac 1380

acctgcatcc caggaccgtg ggggccggtc gtcatttccc tcttgaacat gtgctccgaa 1440

gtataactct gggacctact gcccgtctct ctcttccacc aggttcctgc atgaggagcc 1500

ctgatcaact ggatcaccac tttgcccagc ctctgaacac catgcaccag gcctcaatat 1560

cccagttccc tttggccttt tagttacagg tgaatgctga gaatgtgtca gagacaagtg 1620

cagcagcagc gatggttggt agtatagatc atttactctt cagacaattc ccaaacctcc 1680

attagtccaa gagtttctac atcttcctcc ccagcaagag gcaacgtcaa gtgatgaatt 1740

tccccccttt actctgcctc tgctccccat ttgctagttt gaggaagtga catagaggag 1800

aagccagctg taggggcaag agggaaatgc aagtcacctg caggaatcca gctagatttg 1860

gagaagggaa tgaaactaac attgaatgac taccatggca cgctaaatag tatcttgggt 1920

gccaaattca tgtatccact tagctgcatt ggtccagggc atgtcagtct ggatacagcc 1980

ttacctccag gtagcactta actggtccat tcacctagac tgcaagtaag aagacaaaat 2040

gactgagacc gtgtgcccac ctgaacttat tgtctttact tggcctgagc taaaagcttg 2100

ggtgcaggac ctgtgtaact agaaagttgc ctacttcaga acctccaggg cgtgagtgca 2160

aggtcaaaca tgactggctt ccaggccgac catcaatgta ggaggagagc tgatgtggag 2220

ggtgacatgg gggctgccca tgttaaacct gagtccagtg ctctggcatt gggcagtcac 2280

ggttaaagcc aagtcatgtg tgtctcagct gtttggaggt gatgattttg catcttccaa 2340

gcctcttcag gtgtgaatct gtggtcagga aaacacaagt cctaatggaa cccttagggg 2400

ggaaggaaat gaagattccc tataacctct gggggtgggg agtaggaata aggggccttg 2460

ggcctccata aatctgcaat ctgcaccctc ctcctagaga cagggagatc gtgttctgct 2520

ttttacatga ggagcagaac tgggccatac acatgttcaa gaactagggg agctacctgg 2580

tagcaagtga gtgcagaccc acctcacctt gggggaatct caaactcata ggcctcagat 2640

acacgatcac ctgtcatatc aggtgagcac tggcctgctt ggggagagac ctgggcccct 2700

ccaggtgtag gaacagcaac actcctggct gacaactaag ccaatatggc cctaggtcat 2760

tcttgcttcc aatatgcttg ccactcctta aatgtcctaa tgatgagaaa ctctctttct 2820

gaccaattgc tatgtttaca taacacgcat gtactcatgc atcccttgcc agagcccata 2880

tatgtatgca tatataaaca tagcactttt tactacatag ctcagcacat tgcaaggttt 2940

gcatttaagt t 2951

<210> 7
<211> 3980
<212> DNA
<213> Homo sapiens

<400> 7
tttattttat acggactggc ggcgagagca gctgcagttc gcatctcagg cagtacctag 60

aggagctgcc ggtgcctcct cagaacatct cctgatcgct acccaggacc aggcaccaag 120

gacagggagt cccaggcgca caccccccat tctgggtccc ccaggcccag acccccactc 180

tgccacaggt tgcatcttga cctggtcctc ctgcagaagt ggcccctgtg gtcctgctct 240

gagactcgtc cctgggcgcc cctgcagccc ctttctatga ctccatctgg atttggctgg 300

ctgtggggac gcggtccgag gggcggcctg gctctcagcg tggtggcagc cagctctctg 360

gccaccatgg caaatgctga gatctgaggg gacaaggctc tacagcctca gccaggggca 420

ctcagctgtt gcagggtgtg atggagaaca aagctatgta cctacacacc gtcagcgact 480

gtgacaccag ctccatctgt gaggattcct ttgatggcag gagcctgtcc aagctgaacc 540

tgtgtgagga tggtccatgt cacaaacggc gggcaagcat ctgctgtacc cagctggggt 600

ccctgtcggc cctgaagcat gctgtcctgg ggctctacct gctggtcttc ctgattcttg 660

tgggcatctt catcttagca gtgtccaggc cgcgcagctc ccctgacgac ctgaaggccc 720

tgactcgcaa tgtgaaccgg ctgaatgaga gcttccggga cttgcagctg cggctgctgc 780

aggctccgct gcaagcggac ctgacggagc aggtgtggaa ggtgcaggac gcgctgcaga 840

accagtcaga ctcgttgctg gcgctggcgg gcgcagtgca gcggctggag ggcgcgctgt 900

gggggctgca ggcgcaggcg gtgcagaccg agcaggcggt ggccctgctg cgggaccgca 960

cgggccagca gagcgacacg gcgcagctgg agctctacca gctgcaggtg gagagcaaca 1020

gtagccagct gctgctgagg cgccacgcgg gcctgctgga cgggctggcg cgcagggtgg 1080

gcatcctggg cgaggagctg gccgacgtgg gcggcgtgct gcgcggcctc aaccacagcc 1140

tgtcctacga cgtggccctc caccgcacgc ggctgcagga cctgcgggtg ctggtgagca 1200

acgccagcga ggacacgcgc cgcctgcgcc tggcgcacgt aggcatggag ctgcagctga 1260

agcaggagct ggccatgctc aacgcggtca ccgaggacct gcgcctcaag gactgggagc 1320

actccatcgc actgcggaac atctccctcg cgaaagggcc accgggaccc aaaggtgatc 1380

agggggatga aggaaaggaa ggcaggcctg gcatccctgg attgcctgga cttcgaggtc 1440

tgcccgggga gagaggtacc ccaggattgc ccgggcccaa gggcgatgat gggaagctgg 1500

gggccacagg accaatgggc atgcgtgggt tcaaaggtga ccgaggccca aaaggagaga 1560

aaggagagaa aggagacaga gctggggatg ccagtggcgt ggaggccccg atgatgatcc 1620

gcctggtgaa tggctcaggt ccgcacgagg gccgcgtgga agtgtaccac gaccggcgtt 1680

ggggcaccgt gtgtgacgac ggctgggaca agaaggacgg agacgtggtg tgccgcatgc 1740

tcggcttccg cggtgtggag gaggtgtacc gcacagctcg attcgggcaa ggcactggga 1800

ggatctggat ggatgacgtt gcctgcaagg gcacagagga aaccatcttc cgctgcagct 1860

tctccaaatg gggggtgaca aactgtggac atgccgaaga tgccagcgtg acatgcaaca 1920

gacactgaaa gtgggcagag cccaagttcg gggtcctgca cagagcaccc ttcctgcatc 1980

cctggggtgg ggcacagctc ggggccaccc tgaccatgcc tcgaccacac cccgtccagc 2040

attctcagtc ctcacacctg catcccagga ccgtgggggc cggtcgtcat ttccctcttg 2100

aacatgtgct ccgaagtata actctgggac ctactgcccg tctctctctt ccaccaggtt 2160

cctgcatgag gagccctgat caactggatc accactttgc ccagcctctg aacaccatgc 2220

accaggcctc aatatcccag ttccctttgg ccttttagtt acaggtgaat gctgagaatg 2280

tgtcagagac aagtgcagca gcagcgatgg ttggtagtat agatcattta ctcttcagac 2340

aattcccaaa cctccattag tccaagagtt tctacatctt cctccccagc aagaggcaac 2400

gtcaagtgat gaatttcccc cctttactct gcctctgctc cccatttgct agtttgagga 2460

agtgacatag aggagaagcc agctgtaggg gcaagaggga aatgcaagtc acctgcagga 2520

atccagctag atttggagaa gggaatgaaa ctaacattga atgactacca tggcacgcta 2580

aatagtatct tgggtgccaa attcatgtat ccacttagct gcattggtcc agggcatgtc 2640

agtctggata cagccttacc tccaggtagc acttaactgg tccattcacc tagactgcaa 2700

gtaagaagac aaaatgactg agaccgtgtg cccacctgaa cttattgtct ttacttggcc 2760

tgagctaaaa gcttgggtgc aggacctgtg taactagaaa gttgcctact tcagaacctc 2820

cagggcgtga gtgcaaggtc aaacatgact ggcttccagg ccgaccatca atgtaggagg 2880

agagctgatg tggagggtga catgggggct gcccatgtta aacctgagtc cagtgctctg 2940

gcattgggca gtcacggtta aagccaagtc atgtgtgtct cagctgtttg gaggtgatga 3000

ttttgcatct tccaagcctc ttcaggtgtg aatctgtggt caggaaaaca caagtcctaa 3060

tggaaccctt aggggggaag gaaatgaaga ttccctataa cctctggggg tggggagtag 3120

gaataagggg ccttgggcct ccataaatct gcaatctgca ccctcctcct agagacaggg 3180

agatcgtgtt ctgcttttta catgaggagc agaactgggc catacacatg ttcaagaact 3240

aggggagcta cctggtagca agtgagtgca gacccacctc accttggggg aatctcaaac 3300

tcataggcct cagatacacg atcacctgtc atatcaggtg agcactggcc tgcttgggga 3360

gagacctggg cccctccagg tgtaggaaca gcaacactcc tggctgacaa ctaagccaat 3420

atggccctag gtcattcttg cttccaatat gcttgccact ccttaaatgt cctaatgatg 3480

agaaactctc tttctgacca attgctatgt ttacataaca cgcatgtact catgcatccc 3540

ttgccagagc ccatatatgt atgcatatat aaacatagca ctttttacta catagctcag 3600

cacattgcaa ggtttgcatt taagttaaaa aaaaaaaaaa aaaaaaacta aaggtgaaag 3660

atgccacatt gaacaaacta aattcccaac ccggttctgg caaagaatcc agttatccct 3720

tccatgaaga cgcacataac tctcttactt ggtctttcca ttagggacaa cataagtctt 3780

gttttacatc aaataaaaac aatgttaaaa agtgtgtgaa ccttaaaaat ggaagtctac 3840

tagtttacat acctacttca gaggacatgg aaatgaccat gggcctgcat ttcagggacc 3900

aaagcaaatt aggcctggcc taaaatacat cagacctttt gtaagaaaga atttcaataa 3960

agcaaaaaac atgtcacaag 3980

<210> 8
<211> 1968
<212> DNA
<213> Homo sapiens

<400> 8
tagaggagct gccggtgcct cctcagaaca tctcctgatc gctacccagg accaggcacc 60

aaggacaggg agtcccaggc gcacaccccc cattctgggt cccccaggcc cagaccccca 120

ctctgccaca ggttgcatct tgacctggtc ctcctgcaga agtggcccct gtggtcctgc 180

tctgagactc gtccctgggc gcccctgcag cccctttcta tgactccatc tggatttggc 240

tggctgtggg gacgcggtcc gaggggcggc ctggctctca gcgtggtggc agccagctct 300

ctggccacca tggcaaatgc tgagatctga ggggacaagg ctctacagcc tcagccaggg 360

gcactcagct gttgcagggt gtgatggaga acaaagctat gtacctacac accgtcagcg 420

actgtgacac cagctccatc tgtgaggatt cctttgatgg caggagcctg tccaagctga 480

acctgtgtga ggatggtcca tgtcacaaac ggcgggcaag catctgctgt acccagctgg 540

ggtccctgtc ggccctgaag catgctgtcc tggggctcta cctgctggtc ttcctgattc 600

ttgtgggcat cttcatctta gcagtgtcca ggccgcgcag ctcccctgac gacctgaagg 660

ccctgactcg caatgtgaac cggctgaatg agagcttccg ggacttgcag ctgcggctgc 720

tgcaggctcc gctgcaagcg gacctgacgg agcaggtgtg gaaggtgcag gacgcgctgc 780

agaaccagtc agactcgttg ctggcgctgg cgggcgcagt gcagcggctg gagggcgcgc 840

tatgggggct gcaggcgcag gcggtgcaga ccgagcaggc ggtggccctg ctgcgggacc 900

gcacgggcca gcagagcgac acggcgcagc tggagctcta ccagctgcag gtggagagca 960

acagtagcca gctgctgctg aggcgccacg cgggcctgct ggacgggctg gcgcgcaggg 1020

tgggcatcct gggcgaggag ctggccgacg tgggcggcgt gctgcgcggc ctcaaccaca 1080

gcctgtccta cgacgtggcc ctccaccgca cgcggctgca ggacctgcgg gtgctggtga 1140

gcaacgccag cgaggacacg cgccgcctgc gcctggcgca cgtaggcatg gagctgcagc 1200

tgaagcagga gctggccatg ctcaacgcgg tcaccgagga cctgcgcctc aaggactggg 1260

agcactccat cgcactgcgg aacatctccc tcgcgaaagg gccaccggga cccaaaggtg 1320

atcaggggga tgaaggaaag gaaggcaggc ctggcatccc tggattgcct ggacttcgag 1380

gtctgcccgg ggagagaggt accccaggat tgcccgggcc caagggcgat gatgggaagc 1440

tgggggccac aggaccaatg ggcatgcgtg ggttcaaagg tgaccgaggc ccaaaaggag 1500

agaaaggaga gaaaggagac agagctgggg atgccagtaa ggacattctg ctggggccgt 1560

gggatatggt gttggcacag ggctagctgt cccccgagca gccccataag tttggaggtt 1620

cagaggctgg agccatggct ggggctcaag tgtcaaagga ggctccctac cttttttagg 1680

gctctgctgg tctagcaaga gatgctgata gaccccaggg gcactggcca catttctaga 1740

ggtgtcataa acctggcggt tgtgtgcatg gatctggagg cttcccccgg tcactcgcta 1800

gcccagctgg tataatctct gtgcctcagt gttctcatct ataaaatagg gataacagga 1860

gtctttacct tataaggtca ttgtgaaaat tgaatgagtt aatctgtgta aagtgcttat 1920

gatcatgctg gacacctggt gaggaaaaaa aaaaaaaaaa aaaaaaaa 1968

## Description

The present invention relates to a newly identified protein as marker for the detection of tumors, or as targets for their treatment, particularly of tumors affecting lung, colon, breast and ovary. Also provided are affinity ligands capable of selectively interacting with the newly identified markers, as well as methods for tumor diagnosis and therapy using such ligands.

### Background of the invention

### Tumor markers (or biomarkers)

Tumor markers are substances that can be produced by tumor cells or by other cells of the body in response to cancer. In particular, a protein biomarker is either a single protein or a panel of different proteins that could be used to unambiguously distinguish a disease state. Ideally, a biomarker would have both a high specificity and sensitivity, being represented in a significant percentage of the cases of given disease and not in healthy state.

Biomarkers can be identified in different biological samples, like tissue biopsies or preferably biological fluids (saliva, urine, blood-derivatives and other body fluids), whose collection does not necessitate invasive treatments. Tumor marker levels may be categorized in three major classes on the basis of their clinical use. Diagnostic markers can be used in the detection and diagnosis of cancer. Prognostics markers are indicative of specific outcomes of the disease and can be used to define predictive models that allow the clinicians to predict the likely prognosis of the disease at time of diagnosis. Moreover, prognosis markers are helpful to monitor the patient response to a drug therapy and facilitate a more personalized patient management. A decrease or return to a normal level may indicate that the cancer is responding to therapy, whereas an increase may indicate that the cancer is not responding. After treatment has ended, tumor marker levels may be used to check for recurrence of the tumor. Finally, therapeutic markers can be used to develop tumor-specific drugs or affinity ligand (i.e. antibodies) for a tumor treatment.

Currently, although an abnormal tumor marker level may suggest cancer, this alone is usually not enough to accurately diagnose cancer and their measurement in body fluids is frequently combined with other tests, such as a biopsy and radioscopic examination. Frequently, tumor marker levels are not altered in all of people with a certain cancer disease, especially if the cancer is at early stage. Some tumor marker levels can also be altered in patients with noncancerous conditions. Most biomarkers commonly used in clinical practice do not reach a sufficiently high level of specificity and sensitivity to unambiguously distinguish a tumor from a normal state.

To date the number of markers that are expressed abnormally is limited to certain types/subtypes of cancer, some of which are also found in other diseases. (http://www.cancer.gov/cancertopics/factsheet).

For example, prostate-specific antigen (PSA) levels are often used to screen men for prostate cancer, but this is controversial since elevated PSA levels can be caused by both prostate cancer or benign conditions, and most men with elevated PSA levels turn out not to have prostate cancer.

Another tumor marker, Cancer Antigen 125, (CA 125), is sometimes used to screen women who have an increased risk for ovarian cancer. Scientists are studying whether measurement of CA 125, along with other tests and exams, is useful to find ovarian cancer before symptoms develop. So far, CA 125 measurement is not sensitive or specific enough to be used to screen all women for ovarian cancer. Mostly, CA 125 is used to monitor response to treatment and check for recurrence in women with ovarian cancer. Finally, human epidermal growth factor receptor (HER2) is a marker protein overproduced in about 20% of breast cancers, whose expression is typically associated with a more aggressive and recurrent tumors of this class.

### Routine screening test for tumor diagnosis

Screening tests are a way of detecting cancer early, before there are any symptoms. For a screening test to be helpful, it should have high sensitivity and specificity. Sensitivity refers to the test's ability to identify people who have the disease. Specificity refers to the test's ability to identify people who do not have the disease. Different molecular biology approaches such as analysis of DNA sequencing, small nucleotide polymorphyms, in situ hybridization and whole transcriptional profile analysis have done remarkable progresses to discriminate a tumor state from a normal state and are accelerating the knowledge process in the tumor field. However so far different reasons are delaying their use in the common clinical practice, including the higher analysis complexity and their expensiveness. Other diagnosis tools whose application is increasing in clinics include in situ hybridization and gene sequencing.

Currently, Immuno-HistoChemistry (IHC), a technique that allows the detection of proteins expressed in tissues and cells using specific antibodies, is the most commonly used method for the clinical diagnosis of tumor samples. This technique enables the analysis of cell morphology and the classification of tissue samples on the basis of their immunoreactivity. However, at present, IHC can be used in clinical practice to detect cancerous cells of tumor types for which protein markers and specific antibodies are available. In this context, the identification of a large panel of markers for the most frequent cancer classes would have a great impact in the clinical diagnosis of the disease.

### Anti-cancer therapies

In the last decades, an overwhelming number of studies remarkably contributed to the comprehension of the molecular mechanisms leading to cancer. However, this scientific progress in the molecular oncology field has not been paralleled by a comparable progress in cancer diagnosis and therapy. Surgery and/or radiotherapy are still the main modality of local treatment of cancer in the majority of patients. However, these treatments are effective only at initial phases of the disease and in particular for solid tumors of epithelial origin, as is the case of colon, lung, breast, ovary, prostate and others, while they are not effective for distant recurrence of the disease. In some tumor classes, chemotherapeutic treatments have been developed, which generally relies on drugs, hormones and antibodies, targeting specific biological processes used by cancers to grow and spread. However, so far many cancer therapies had limited efficacy due to severity of side effects and overall toxicity. Indeed, a major effort in cancer therapy is the development of treatments able to target specifically tumor cells causing limited damages to surrounding normal cells thereby decreasing adverse side effects. Recent developments in cancer therapy in this direction are encouraging, indicating that in some cases a cancer specific therapy is feasible. In particular, the development and commercialization of humanized monoclonal antibodies that recognize specifically tumor-associated markers and promote the elimination of cancer is one of the most promising solution that appears to be an extremely favorable market opportunity for pharmaceutical companies. However, at present the number of therapeutic antibodies available on the market or under clinical studies is very limited and restricted to specific cancer classes. So far licensed monoclonal antibodies currently used in clinics for the therapy of specific tumor classes show only a partial efficacy and are frequently associated with chemotherapies to increase their therapeutic effect. Administration of Trastuzumab (Herceptin), a commercial monoclonal antibody targeting HER2in conjunction with Taxol adjuvant chemotherapy induces tumor remission in about 42% of the cases (1). Bevacizumab (Avastin) and Cetuximab (Erbitux) are two monoclonal antibodies recently licensed for use in humans, targeting the endothelial and epithelial growth factors respectively that, combined with adjuvant chemotherapy, proved to be effective against different tumor diseases. Bevacizumab proved to be effective in prolonging the life of patients with metastatic colorectal, breast and lung cancers. Cetuximab demostrated efficacy in patients with tumor types refractory to standard chemotherapeutic treatments (1).

In summary, available screening tests for tumor diagnosis are uncomfortable or invasive and this sometimes limits their applications. Moreover tumor markers available today have a limited utility in clinics due to either their incapability to detect all tumor subtypes of the defined cancers types and/or to distinguish unambiguously tumor vs. normal tissues. Similarly, licensed monoclonal antibodies combined with standard chemotherapies are not effective against the majority of cases. Therefore, there is a great demand for new tools to advance the diagnosis and treatment of cancer.

### Experimental approaches commonly used to identify tumor markers

Most popular approaches used to discover new tumor markers are based on genome-wide transcription profile or total protein content analyses of tumor. These studies usually lead to the identification of groups of mRNAs and proteins which are differentially expressed in tumors. Validation experiments then follow to eventually single out, among the hundreds of RNAs/proteins identified, the very few that have the potential to become useful markers. Although often successful, these approaches have several limitations and often, do not provide firm indications on the association of protein markers with tumor. A first limitation is that, since frequently mRNA levels not always correlate with corresponding protein abundance (approx. 50% correlation), studies based on transcription profile do not provide solid information regarding the expression of protein markers in tumor (2, 3, 4, 5). A second limitation is that neither transcription profiles nor analysis of total protein content discriminate post-translation modifications, which often occur during oncogenesis. These modifications, including phosphorylations, acetylations, and glycosylations, or protein cleavages influence significantly protein stability, localization, interactions, and functions (6).

As a consequence, large scale studies generally result in long lists of differentially expressed genes that would require complex experimental paths in order to validate the potential markers. However, large scale genomic/proteomic studies reporting novel tumor markers frequently lack of confirmation data on the reported potential novel markers and thus do not provide solid demonstration on the association of the described protein markers with tumor.

### Approach used to identify the protein marker included in the present invention

The approach that we used to identify a tumor marker is based on an innovative immuno-proteomic technology. In essence, a library of recombinant human proteins has been produced from E. coli and it is used to generate polyclonal antibodies against each of the recombinant proteins.

The screening of the antibodies library on Tissue microarrays (TMAs) carrying clinical samples from different patients affected by the tumor under investigation leads to the identification of specific tumor marker proteins. Therefore, by screening TMAs with the antibody library, the tumor markers are visualized by IHC, the classical technology applied in all clinical pathology laboratories. Since TMAs also include healthy tissues, the specificity of the antibodies for the tumors can be immediately appreciated and information on the relative level of expression and cellular localization of the markers could be obtained. In our approach the markers are subjected to a validation process consisting in a molecular and cellular characterization. Altogether, the selective detection of the marker protein in tumor samples and the subsequent validation experiments lead to an unambiguous confirmation of the marker identity and confirm its association with defined tumor classes. Moreover this experimental process provides an indication of the possible use of the protein as tools for diagnostic or therapeutic intervention. For instance, a protein showing a cell surface localization could be both diagnostic and therapeutic marker, against which both chemical and antibody therapies can be developed. Differently, a marker showing a cytoplasmic localization could be more likely considered for the development of tumor diagnostic tests and chemotherapy/small molecules treatments.

### Summary of the invention

The present invention provides new means for the detection and treatment of breast, colon, lung and ovary tumors, based on the identification of Scavenger receptor class A member 5 (SCARA 5) marker specific for these tumor types.

In preferred embodiments, the invention provides the use of SCARA5 as marker or target for breast, colon, lung, ovary tumors.

The invention also provides a method for the diagnosis of these cancer types, comprising a step of detecting the above-identified marker in a biological sample, e.g. in a tissue sample of a subject suspected of having or at risk of developing malignancies or susceptible to cancer recurrences.

In addition, the tumor marker identifies a novel target for affinity ligands which can be used for therapeutic applications. Also provided are affinity ligands, particularly antibodies, capable of selectively interacting with the newly identified protein marker.

### State of the art

Despite the involment of SCARA5 in tumor has been partially investigated, so far no previous evidence clearly documents the association of SCARA5 with breast, lung, ovary and colon tumors.

Recently, SCARA 5 has been studied in Hepatocellular Carcinoma (HCC) (8). In this tumor type SCARA5 has been reported to be a tumor suppressor gene, whose espression was frequently downregulated as a result of promoter hypermethylation and allelic imbalance. Furthermore, SCARA5 knockdown via RNA interference markedly enhanced HCC cell growth in vitro, colony formation in soft agar, and invasiveness, tumorigenicity, and lung metastasis in vivo. By contrast, SCARA5 overexpression suppressed these malignant behaviors. Moreover, SCARA5 was found to physically associate with focal adhesion kinase (FAK), a non-receptor tyrosine kinase, and modulate tyrosine phosphorylation. FAK is known to be important in the regulation of focal adhesion dynamics and disassembly during cell migration. It is activated in a range of tumor cells, and its increased activity correlates with the malignancy and invasiveness of human HCC and other tumors. The interaction of SCARA5 with FAK inhibits the tyrosine phosphorylation cascade of the FAK-Src-Cas signaling pathway. Conversely, silencing SCARA5 stimulated the signaling pathway via increased phosphorylation of certain tyrosine residues of FAK, Src, and p130Cas; it was also associated with activation of MMP9, a tumor metastasis-associated enzyme. Taken together, these experimental data indicate that SCARA5 overexpression inhibits tumorigenicity, cell invasion, and metastasis; on the contrary, SCARA5 knockdown enhances tumorigenicity, cell invasion, and tumor metastasis in vivo via activation of the FAK signaling pathway.

SCARA5 has been included in some patent applications on specific types of cancer.

A European patent application (EP2159291A1, publication date 29.01.2010) reports SCARA5 as a non-priority target in a set of 30 signature genes, selected on the basis of transcription profile analysis in breast cancer tumors and claimed as diagnosis tool for these cancer types. Another international application (WO2008104543A2) includes Scara5 within a list of approximately 300 differentially expressed genes in bone tissue metastasis of patients affected by breast cancer. There is no mention to a possible use of these genes for the diagnosis of breast tumor but they are claimed as predictive of occurrence of metastasis of breast cancer. In both patent applications, evidences are limited to RNA analysis and no experimental confirmation is provided on the expression of SCARA5 protein in these tumor types. Moreover, the data support the diagnostic/predictive value of a signature/cluster of genes, while no evidence is provided on the possibility to exploit the transcript level of Scara5, as single gene, for the indicated purpose.

SCARA 5 is included in a patent application (WO2009093213A2, international publication date 23.09.2009) disclosing five clusters of genes useful for predicting or diagnosing outcome of brain tumor treatment. In this application, SCARA5 is reported as a non-priority member of one of five clusters of genes whose high transcription level indicates a better outcome to chemo-radiotherapy treatment of brain tumors. Also in this case, data are limited to RNA expression in brain tumor samples with respect to the normal tissues, while no evidence of protein differential expression is reported.

Finally SCARA5 is mentioned in a US patent application (US20090047689A1 filed 2008-06-20) related to autoantigen biomarkers for early diagnosis of lung adenocarcinoma. The application is focused on the identification of a recognition profile of serum from patients affected by lung adenocarcinoma which is proposed as a tool for tumor diagnosis. A panel of 133 proteins (autoantigens) was identified, showing higher reactivity with serum IgGs from patients affected by lung adenocarcinoma, compared to heathy individuals. In this long list of proteins is included SCARA5. However, there is no evidence supporting the use of individual autoantigens for the diagnosis. Moreover, no data are given on the over-expression of SCARA5 in tumor samples. Finally, the applications is not related to the identification of SCARA5 in tumor samples.

None of the cited publications provide sufficient evidences that expression of SCARA5 protein is associated with tumor, in that: i) in hepatocellular carcinoma, high SCARA5 expression is inversely correlated with a tumor state; ii) patent application data reporting the differential expression of Scara5 in breast and lung cancers are only based on transcription profile analysis of tumor versus healthy states, but no supportive data are provided confirming the presence of SCARA5 protein in these tumors; as reported in the previous section, frequently there is no correlation between mRNA and protein expression level, iii) the patent application describing SCARA5 as autoantigen, able to induce antibodies in patients affected by lung cancer, does not provide evidences on the over-expression of SCARA5 in tissues of these tumor type. Differently, we describe SCARA5 as a tumor biomarker and report the possibility of using a diagnostic method based on the direct detection of this protein; iv) none of the cited patent applications (reporting either increase of Scara5 transcript or elicitation of antibodies in patients) shows the diagnostic property of SCARA5 when used individually; v) finally, none of the reported studies provide indication on the possible use of SCARA5 as a target for therapeutic intervention.

### Disclosure of the invention

The present invention is based on the surprising finding that antibodies specific for Scara5 are able to specifically stain breast, colon, lung, ovary tumor tissues from patients, while negative or very poor staining is observed in normal lung tissues from the same patients. These antibodies have been found to specifically bind to a protein for which no previous association with tumor has been reported.

According to the present invention SCARA5 is provided as a protein marker for breast, colon, lung and ovary tumors and in general for cancers of these types. As described below, an antibody generated towards the SCARA5 protein shows a selective immunoreactivity in histological preparation of breast, colon, lung and ovary cancer tissues which indicates the presence of SCARA5 in these cancer samples and makes SCARA5 protein and its antibody highly interesting tools for specifically distinguishing these cancer types from a normal state. Moreover, an antibody generated against SCARA5 is able to specifically recognize the protein on the surface of cancer cell lines, indicating that this proteins could be developed as a target for anti-cancer therapies.

Hence, in a first aspect, the invention provides a marker for breast, colon, lung and ovary tumors which is selected from:
(i) SCARA5 protein, in one of its isoforms SEQ ID NO:1, SEQ ID NO:2, SEQ ID NO:3 or SEQ ID NO:4, or a different isoform having sequence identity of at least 80%, preferably at least 90%, more preferably at least 95% to SEQ ID NO:1, SEQ ID NO:2, SEQ ID NO:3 or SEQ ID NO:4
(ii) a nucleic acid molecule containing a sequence coding for a SCARA5 protein, said encoding sequence being preferably SEQ ID NO:5, SEQ ID NO:6, SEQ ID NO:7 or SEQ ID NO:8;

As used herein the "% amino acid sequence identity" with respect to the marker protein sequences identified herein indicates the percentage of amino acid residues in a protein variant or isoform, or in a portion thereof, that are identical to the amino acid residues in the specific marker sequence, after aligning the sequences and introducing gaps, if necessary, to achieve the maximum percent sequence identity, and not considering any conservative substitution as part of the sequence identity.

Scavenger receptor class A member 5 (SCARA 5; synonyms: Tesr; NET33; FLJ23907; MGC45780; GeneID: ENSG00000168079; Transcript IDs: ENST00000354914, ENST00000301906, ENST00000380385, BC033153; Protein IDs: ENSP00000346990, ENSP00000301906, ENSP00000369746, AAH33153.1) is a plasma membrane protein acting as a ferritin receptor that mediates non-transferrin-dependent delivery of iron. It mediates cellular uptake of ferritin-bound iron by stimulating ferritin endocytosis from the cell surface with consequent iron delivery within the cell. Delivery of iron to cells by ferritin is required for the development of specific cell types, suggesting the existence of cell type-specific mechanisms of iron traffic in organogenesis, which alternatively utilize transferrin or non-transferrin iron delivery pathways. (7)

A further aspect of this invention is a method of screening a tissue sample for malignancy, which comprises determining the presence in said sample of the above-mentioned tumor marker. This method includes detecting either the marker protein, e.g. by means of labeled monoclonal or polyclonal antibodies that specifically bind to the target protein, or the respective mRNA, e.g. by means of polymerase chain reaction techniques such as RT-PCR. The methods for detecting proteins in a tissue sample are known to one skilled in the art and include immunoradiometric, immunoenzymatic or immunohistochemical techniques, such as radioimmunoassays, immunofluorescent assays or enzyme-linked immunoassays. Other known protein analysis techniques, such as polyacrylamide gel electrophoresis (PAGE), Western blot or Dot blot are suitable as well. Preferably, the detection of the protein marker is carried out with the immune-histochemistry technology, particularly by means of High Through-Put methods that allow the analyses of the antibody immune-reactivity simultaneously on different tissue samples immobilized on a microscope slide. Briefly, each Tissue Micro Array (TMA) slide includes tissue samples suspected of malignancy taken from different patients, and an equal number of normal tissue samples from the same patients as controls. The direct comparison of samples by qualitative or quantitative measurement, e.g. by enzimatic or colorimetric reactions, allows the identification of tumors.

In one embodiment, the invention provides a method of screening a sample of lung, colon, breast or ovary tissue for malignancy, which comprises determining the presence in said sample of the SCARA5 protein tumor marker, variants or isoforms thereof as described above.

A further aspect of the invention is a method *in vitro* for determining the presence of a lung, colon, breast or ovary tumor in a subject, which comprises the steps of:
- providing a sample of the tissue suspected of containing tumor cells;
- determining the presence of a SCARA5 tumor marker in said tissue sample by detecting the expression of the marker protein or the presence of the respective mRNA transcript;
wherein the detection of the tumor marker in the tissue sample is indicative of the presence of tumor in said subject.

The methods and techniques for carrying out the assay are known to one skilled in the art and are preferably based on immunoreactions for detecting proteins and on PCR methods for the detection of mRNAs. The same methods for detecting proteins or mRNAs from a tissue sample as disclosed above can be applied.

A further aspect of this invention is the use of the SCARA5 tumor marker herein provided as target for the identification of candidate antitumor agents. Accordingly, the invention provides a method for screening compounds which comprises contacting cells expressing SCARA5 protein with the test compound and determining the binding of said compound to said tumor-associated protein. In addition, the ability of the test compound to modulate the activity of each target molecule can be assayed.

A further aspect of the invention is an antibody or a fragment thereof, which is able to specifically recognize and bind to one of the tumor-associated proteins described above. The term "antibody" as used herein refers to any type of immunoglobulins, including IgG, IgM, IgA, IgD and IgE. Such antibodies may include polyclonal, monoclonal, chimeric, single chain, antibodies or fragments such as Fab or scFv. The antibodies may be of various origin, including human, mouse, rat, rabbit and horse, or chimeric antibodies. The production of antibodies is well known in the art. For the production of antibodies in experimental animals, various hosts including goats, rabbits, rats, mice, and others, may be immunized by injection with polypeptides of the present invention or any fragment or oligopeptide or derivative thereof which has immunogenic properties or forms a suitable epitope. Monoclonal antibodies may be produced following the procedures described in Kohler and Milstein, Nature 265:495 (1975) or other techniques known in the art.

The antibodies to the tumor markers of the invention can be used to detect the presence of the marker in histologic preparations or to distinguish tumor cells from normal cells. To that purpose, the antibodies may be labeled with radiocative, fluorescent or enzyme labels.

In addition, the antibodies can be used for treating proliferative diseases by modulating, e.g. inhibiting or abolishing the activity of a target protein according to the invention. Therefore, in a further aspect the invention provides the use of antibodies to SCARA5 protein for the preparation of a therapeutic agent for the treatment of proliferative diseases. For use in therapy, the antibodies can be formulated with suitable carriers and excipients, optionally with the addition of adjuvants to enhance their effetcs.

A further aspect of the invention relates to a diagnostic kit containing suitable means for detection, in particular SCARA5 polypeptides or polynucleotides, antibodies or fragments or derivatives thereof described above, reagents, buffers, solutions and materials needed for setting up and carrying out the immunoassays, nucleic acid hybridization or PCR assays described above. Parts of the kit of the invention can be packaged individually in vials or bottles or in combination in containers or multicontainer units.

### Description of the Figures

**Figure 1****. Analysis of purified SCARA5 recombinant protein expressed in *E.coli***
   Left panel: Comassie staining of purified His-tag SCARA5 fusion protein expressed in *E*. *coli* separated by SDS-PAGE. Arrow marks the protein band of the expected size. Molecular weight markers are reported on the left.
**Figure 2****. Staining of breast tumor TMAs with anti-SCARA5 antibodies**
   Examples of TMA of breast tumor (lower panel) and normal tissue samples (upper panel) stained with anti- SCARA5 antibodies. The antibody stains specifically tumor cells (in dark gray).
**Figure 3****. Staining of colon tumor TMAs with anti-SCARA5 antibodies**
   Examples of TMA of colon tumor (lower panel) and normal tissue samples (upper panel) stained with anti- SCARA5 antibodies. The antibody stains specifically tumor cells (in dark gray).
**Figure 4****. Staining of lung tumor TMAs with anti-SCARA5 antibodies**
   Examples of TMA of lung tumor (lower panel) and normal tissue samples (upper panel) stained with anti- SCARA5 antibodies. The antibody stains specifically tumor cells (in dark gray).
**Figure 5****. Staining of ovary tumor TMAs with anti-SCARA5 antibodies**
   Examples of TMA of ovary tumor (lower panel) and normal tissue samples (upper panel) stained with anti- SCARA5 antibodies. The antibody stains specifically tumor cells (in dark gray).

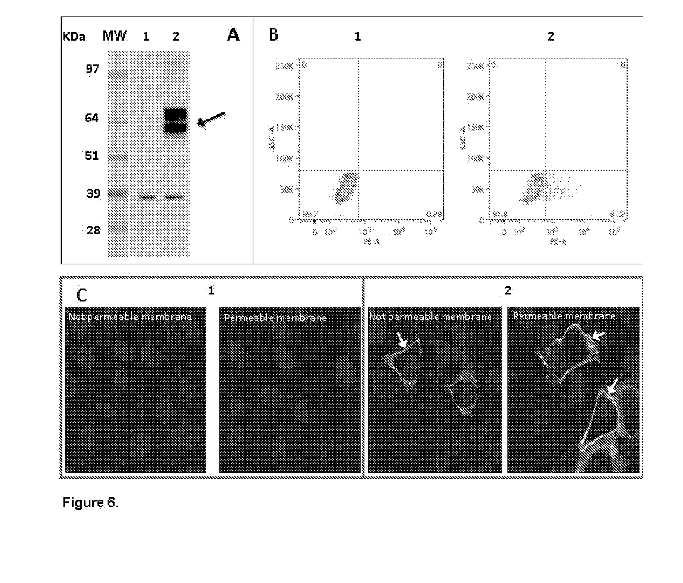

**Figure 6****. SCARA5 expression and localization in transiently transfected HeLa cells. A) Western blot analysis of SCARA5 expression in** total protein extracts from HeLa cells (corresponding to 2x105 cells) transfected with the empty vector pcDNA3 (lane 1) or with the plasmid construct encoding the SCARA5 gene (lane 2) stained with a specific antibody. Two bands of approximately 60 and 70KDa were visible in HeLa cells transfected with plasmid expressing the SCARA5 495 amino acid-protein isoform, while the same bands were not visible in HeLa cells transfected with the empty pcDNA3 plasmid. The 60KDa protein species shows the expected molecular mass, while the 70KDa species likely corresponds to a SCARA5 form carrying post-translational modifications (e.g. glycolyslation). Arrow marks the expected SCARA5 protein band. Molecular weight markers are reported on the left. B) Flow cytometry analysis of SCARA5 surface localization in HeLa cells transfected with the plasmid construct encoding the SCARA5 gene (panel 2), compared to control cells transfected with the empty vector pcDNA3 (panel 1) . X axys, Fluorescence scale; Y axys, Side Scatter scale C) Confocal microscopy analysis of SCARA5 surface localization in HeLa cells transfected with the with the plasmid construct encoding the SCARA5 gene (2) or with empty vector pcDNA3, as control (1) and stained with anti-SCARA5 antibodies and DAPI to visualize the nuclei. Left and right panels represent the surface and intracellular cell staining, respectively. The SCARA5 specific staining accumulated at the surface of transfected cells while no staining was detected in control HeLa cells (marked by arrows).

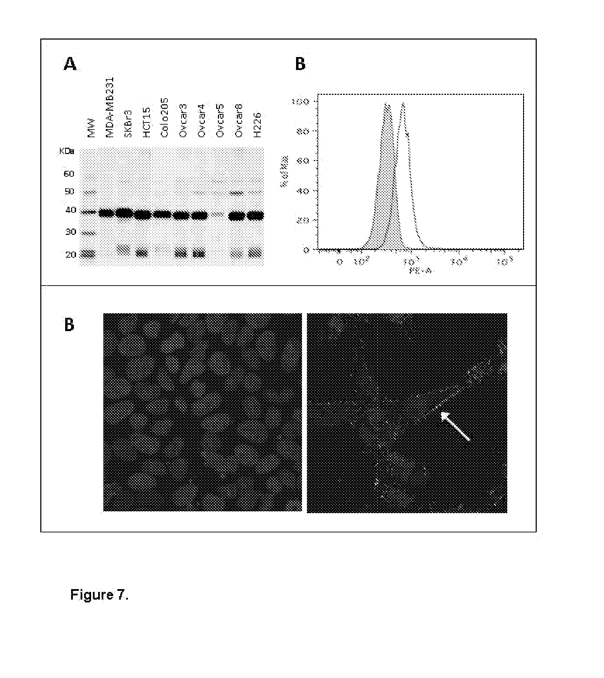

**Figure 7****. Expression and localization of SCARA5 in tumor cell lines** A) Western blot analysis of SCARA5 expression. Total protein extracts (corresponding to 2x10⁵ cells) from breast (MDA-MB231 and SKBr3), colon (HCT15 and Colo205), ovary (Ovcar3, Ovcar4, Ovcar5, Ovcar8) and lung (H226) tumor cell lines were separated by SDS-PAGE, transferred onto nitrocellulose membranes and probed with anti-SCARA5 antibodies. Molecular weight markers are reported on the left. B) Flow cytometry analysis of SCARA5 surface localization in the Ovcar8 cell line stained with the anti-SCARA5 (white peak) or unrelated antibodies (grey peak). X axys, Fluorescence scale; Y axys, Cells (expressed as % relatively to major peaks). C) Confocal microscopy analysis of SCARA5 surface localization Ovcar8 tumor cell line stained with anti-SCARA5 (right panel) or irrelevant antibodies (left panel) and DAPI to visualize the nuclei. Arrow marks the SCARA5 staining visible at the cell surface.
**Figure 8****. Detection of SCARA5 in lung tumor tissue homogenates by immunoblot.** Immunoblot analysis of tissue homogenates from biopsies of lung tumor (lanes 4, 5, 6) and corresponding normal samples (lanes 1, 2, 3) stained with an anti-SCARA antibody. Molecular weight markers are reported on the left. Different protein species ranging from 40 to 20KDa were specifically detected by the antibody, compatible with annotated SCARA5 isoforms.

### EXAMPLES

### Example 1. Generation of recombinant SCARA5 and anti-SCARA5 antibodies to detect the expression of SCARA5 in tumor samples

### Methods

The entire coding region or suitable fragments of the Scara 5 gene encoding the target protein, were designed for cloning and expression using bioinformatic tools with the human genome sequence as template (Lindskog M et al (2005). The leader sequence for secretion was replaced with the ATG codon to drive the expression of the recombinant proteins in the cytoplasm of E. coli. For cloning, genes were PCR-amplified from cDNAs mixtures generated from pools of total RNA derived from Human testis, Human placenta, Human bone marrow, Human fetal brain, using specific primers. Clonings were designed so as to fuse a 10 histidine tag sequence at the 3' end, annealed to in house developed vectors, derivatives of vector pSP73 (Promega) adapted for the T4 ligation independent cloning method (9) and used to transform *E. coli* NovaBlue cells recipient strain. *E. coli* tranformants were plated onto selective LB plates containing 100 µg/ml ampicillin (LB Amp) and positive E.coli clones were identified by restriction enzyme analysis of purified plasmid followed by DNA sequence analysis. For expression, plasmids were used to transform BL21-(DE3) *E.coli* cells and BL21-(DE3) E. coli cells harbouring the plasmid were inoculated in ZYP-5052 growth medium (10) and grown at 37°C for 24 hours. Afterwards, bacteria were collected by centrifugation, lysed into B-Per Reagent containing 1 mM MgCl2, 100 units DNAse I (Sigma), and 1 mg/ml lysozime (Sigma). After 30 min at room temperature under gentle shaking, the lysate was clarified by centrifugation at 30.000 g for 40 min at 4°C. Proteins were purified from the inclusion bodies by resuspending the pellet coming from lysate centrifugation in 40 mM TRIS-HCl, 1 mM TCEP {Tris(2-carboxyethyl)-phosphine hydrochloride, Pierce} and 6M guanidine hydrochloride, pH 8 and performing an IMAC in denaturing conditions. Briefly, the resuspended material was clarified by centrifugation at 30.000 g for 30 min and the supernatant was loaded on 0.5 ml columns of Ni-activated Chelating Sepharose Fast Flow (Pharmacia). The column was washed with 50 mM TRIS-HCl buffer, 1 mM TCEP, 6M urea, 60 mM imidazole, 0.5M NaCl, pH 8. Recombinant proteins were eluted with the same buffer containing 500 mM imidazole. Proteins were analysed by SDS-Page and their concentration was determined by Bradford assay using the BIORAD reagent (BIORAD) with a bovine serum albumin standard according to the manufacturer's recommendations.

To generate antisera, the purified SCARA5 recombinant proteins were used to immunize CD1 mice (6 week-old females, Charles River laboratories, 5 mice per group) intraperitoneally, with 3 protein doses of 20 micrograms each, at 2 week-interval. Freund's complete adjuvant was used for the first immunization, while Freund's incomplete adjuvant was used for the two booster doses. Two weeks after the last immunization animals were bled and sera collected from each animal was pooled.

### Results

Gene fragments of the expected size were obtained by PCR from cDNA generated from pools of total RNA derived from Human testis, Human placenta, Human bone marrow, Human fetal brain,using primers specific for Scara5 gene.

A fragment of the transcript ENST00000301906 encoding a protein of 318 residues, corresponding to the amino acid region from 40 to 357 of ENSP00000301906 sequence was obtained.

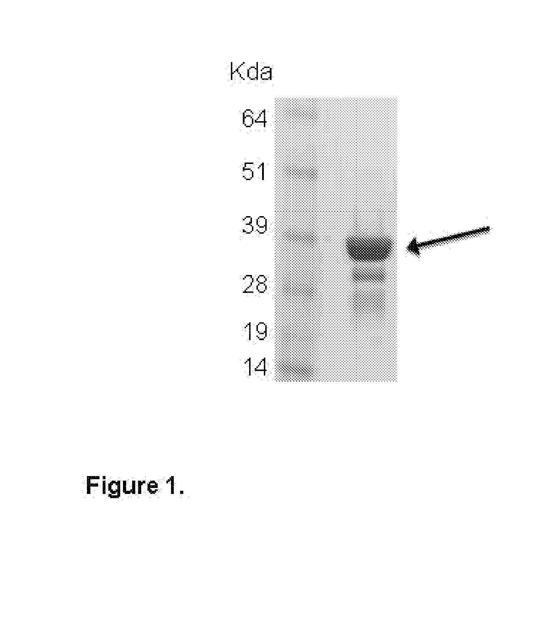

A clone encoding the correct amino acid sequence was identified and, upon expression in *E*. *coli,* a protein of the correct size was produced and subsequently purified using affinity chromatography (Figure 1).

### Example 2. Tissue profiling by immune-histochemistry

### Methods

The analysis of the antibody capability to recognize their target proteins in tumor samples was carried out by Tissue Micro Array (TMA), a miniaturized immuno-histochemistry technology suitable for HTP analysis that allows to analyse the antibody immuno-reactivity simultaneously on different tissue samples immobilized on a microscope slide.

Since the TMAs include both tumor and healthy tissues, the specificity of the antibodies for the tumors can be immediately appreciated. The use of this technology, differently from approaches based on transcription profile, has the important advantage of giving a first hand evaluation on the potential of the markers in clinics. Conversely, since mRNA levels not always correlate with protein levels (approx. 50% correlation), studies based on transcription profile do not provide solid information regarding the expression of protein markers.

A tissue microarray was prepared containing formalin-fixed paraffin-embedded cores of human tissues from patients affected by breast, colon, lung and ovary cancers and corresponding normal tissues as controls and subsequently analyzed using the specific antibody sample. For each tumor class the TMA design consisted in 10 pathological and 10 normal tissue samples from 5 well pedigreed patients (equal to two tumor samples and 2 normal tissues from each patient) to identify promising target molecules differentially expressed in cancer and normal cells. The direct comparison between tumor and normal tissues of each patient allowed the identification of antibodies that stain specifically tumor cells and provided indication of target expression in lung tumor.

All formalin fixed, paraffin embedded tissues used as donor blocks for TMA production were selected from the archives at the IEO (Istituto Europeo Oncologico, Milan). Corresponding whole tissue sections were examined to confirm diagnosis and tumor classification, and to select representative areas in donor blocks. Normal tissues were defined as microscopically normal (non- neoplastic) and were generally selected from specimens collected from the vicinity of surgically removed tumors. The TMA production was performed essentially as previously described (11, 12). Briefly, a hole was made in the recipient TMA block. A cylindrical core tissue sample (1 mm in diameter) from the donor block was acquired and deposited in the recipient TMA block. This was repeated in an automated tissue arrayer "Galileo TMA CK 3500" della dita BioRep (Milan) until a complete TMA design was produced. TMA recipient blocks were baked at 42 <0>C for 2 h prior to sectioning. The TMA blocks were sectioned with 2-3 mm thicknes using a waterfall microtome (Leica), and placed onto poli-L-lysinated glass slides for immunohistochemical analysis. For automated immunohistochemistry, glass slides were incubated for 30' min in 60°C, de-paraffinized in xylene (2 x 15 min) using the Bio-Clear solution (Midway. Scientific, Melbourne, Australia), and re-hydrated in graded alcohols. For antigen retrieval, slides were immersed 0.01 M Na-citrate buffer, pH 6.0 at 99°C for 30 min Slides were placed in the Autostainer (R) (DakoCytomation) and endogenous peroxidase was initially blocked with 3% H2O2, for 5 min. Slides were then blocked in Dako Cytomation Wash Buffer containing 5% Bovine serum albumin (BSA) and subsequently incubated with mouse antibodies for 30' (dilution 1:200 in Dako Real ™ dilution buffer). After washing with DakoCytomation wash buffer, slides were incubated with the goat anti-mouse peroxidase conjugated Envision(R) for 30 min each at room temperature (DakoCytomation). Finally, diaminobenzidine (DakoCytomation) was used as chromogen and Harris hematoxylin (Sigma- Aldrich) was used for counterstaining. The slides were mounted with Pertex(R) (Histolab).

The staining results have been evaluated by a trained pathologist at the light microscope, and scored according to both the percentage of immunostained cells and the intensity of staining. The individual values and the combined score (from 0 to 300) were recorded in a custom-tailored database. Digital images of the immunocytochemical findings have been taken at a Leica DM LB light microscope, equipped with a Leica DFC289 color camera.

### Results

A TMA design was obtained, including tumor tissue samples and normal tissues, derived from 5 patients affected by breast, colon, lung and ovary tumor. The results from tissue profiling showed that the antibodies specific for the recombinant proteins (see Example 1) are strongly immunoreactive on tissues from each of the four cancers, while no or poor reactivity was detected in normal tissues, indicating the presence of the target proteins in lung tumors. In most samples, the antibody staining accumulated at the plasma membrane of tumor cells. Based on this finding, the detection of SCARA5 protein in tumor tissue samples can be associated with lung breast, colon, lung and ovary tumors. Moreover, the SCARA5 localization at the plasma membrane makes this protein as a suitable target for therapies.

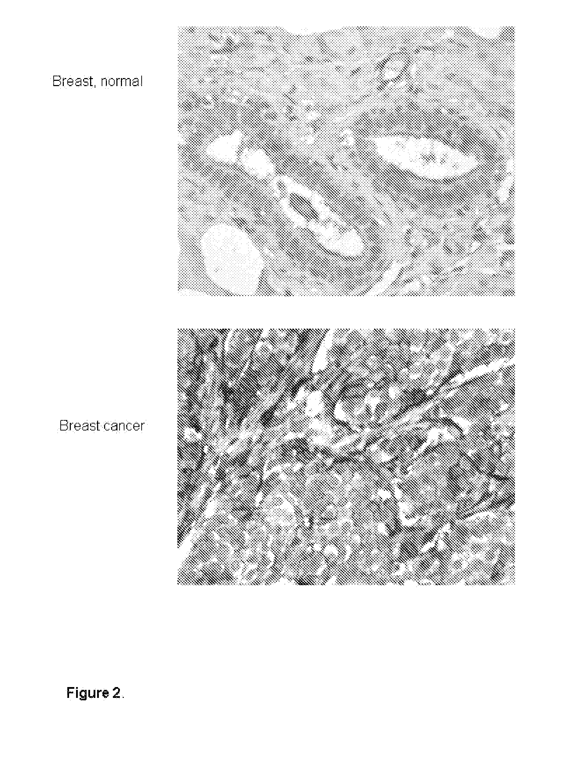

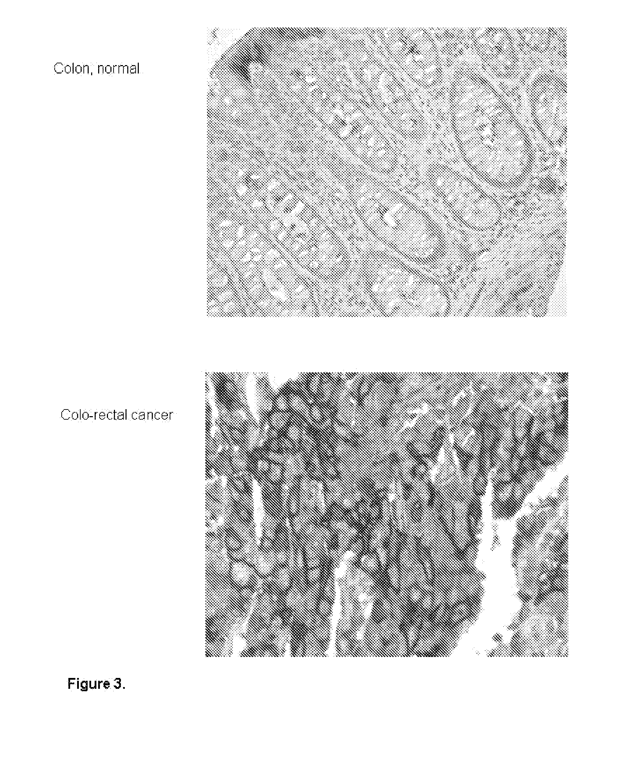

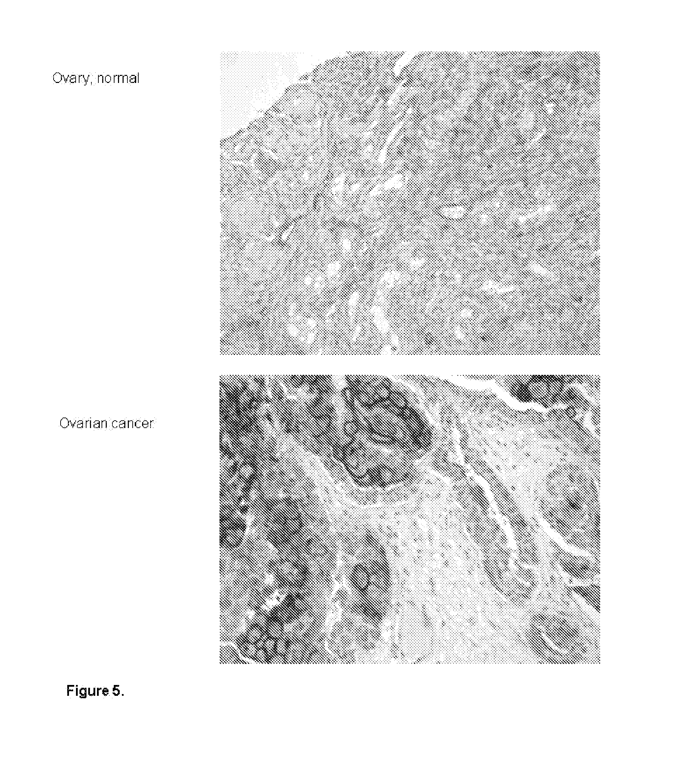

The capability of target-specific antibodies to stain the tumor tissues is summarized in Table 1. Representative examples of microscopic enlargements of tissue samples stained by the anti-SCARA5 antibody are reported in Figures 2-5.

The table reports the number of patients, out of the five screened, whose tumor tissue samples showed positive staining with the SCARA5-specific antibodies.

| **Table 1**. Number of patients whose tumor tissues showed positive immuno-histochemistry staining with the anti-SCARA5 antibodies | |
|---|---|
| **Tumor** | **Number of positive patients** |
| Breast | 4/5 |
| Colon | 3/5 |
| Lung | 4/5 |
| Ovary | 2/5 |

### Example 3. Confirmation of the marker association with the tumor/s by expanded IHC analysis

### Methods

The association of each protein with the indicated tumors was further confirmed on a larger collection of clinical samples for each tumor. To this aim, a tissue microarray was prepared for each of the four tumor classes containing 100 formalin-fixed paraffin-embedded cores of human tissues from 50 patients (equal to two tissue samples from each patient). The TMAs were stained with the SCARAS-antibodies, using the previously reported procedure. The staining results were evaluated, as above described, by a trained pathologist at the light microscope.

### Results

Four TMA designs were obtained, for each of the four tumors, representing tissue samples from 50 patients. The results from tissue analysis showed that the anti-SCARA5 antibodies are strongly immune-reactive on a significant percentage of tissues from breast, colon, lung and ovary tumors indicating that the SCARA5 protein is are selectively detected in these tumor types. This finding confirms a strong association of SCARA5 marker with the indicated tumors. Table 2 reports the frequency of patients whose tumor tissue samples that showed positive IHC staining on the TMA.

| **Table 2**. Frequency of patients whose tumor tissues showed positive immuno-histochemistry staining with the anti-SCARA5 antibodies on the expanded TMA analysis | |
|---|---|
| **Tumor** | **Percentage of positive patients** |
| **Breast** | 38 |
| **Colon** | 56 |
| **Lung** | 42 |
| **Ovary** | 34 |

### Example 4. Expression and localization of SCARA5 protein in transfected mammalian cells

### Methods

SCARA5 expression was assessed by Western blot analysis on total protein extracts from eukaryotic cells transiently transfected with plasmid constructs containing the complete coding sequences of the genes encoding SCARA5 protein. Examples of this type of experiments are given for HeLa cells transfected with plasmid pcDNA3.1 in which one the annotated SCARA5 isoform (corresponding to Transcript ID ENST00000301906 and encoding a protein of 495 aminoacids) was cloned.

To this aim, cDNA were generated from pools of total RNA derived from Human testis, Human placenta, Human bone marrow, Human fetal brain, in reverse transcription reactions and the entire SCARA 5 coding region was PCR-amplified with specific primers pairs. PCR products were cloned into plasmid pcDNA3 (Invitrogen). HeLa cells were grown in DMEM-10% FCS supplemented with 1 mM Glutamine were transiently transfected with preparation of the resulting plasmid and with the empty vector as negative control using the Lipofectamine-2000 transfection reagent (Invitrogen). After 48 hours, cells were collected, lysed with PBS buffer containing 1% Triton X100 and expression of target proteins was assessed by Western blot analysis on total cell extracts (corresponding to 2x10⁵ cells) using anti-SCARA5 antibodies. Western blot was performed by separation of the protein extracts on pre-cast SDS-PAGE gradient gels (NuPage 4-12% Bis-Tris gel, Invitrogen) under reducing conditions, followed by electro-transfer to nitrocellulose membranes (Invitrogen) according to the manufacturer's recommendations. The membranes were blocked in blocking buffer composed of 1x PBS-0.1% Tween 20 (PBST) added with 10% dry milk, for 1 h at room temperature, incubated with the antibody diluted 1:2500 in blocking buffer containing 1% dry milk and washed in PBST-1%. The secondary HRP-conjugated antibody (goat anti-mouse immunoglobulin/HRP, Perkin Elmer) was diluted 1:5000 in blocking buffer and chemiluminescence detection was carried out using a Chemidoc-IT UVP CCD camera (UVP) and the Western lightning^{™} cheminulescence Reagent Plus (Perkin Elmer), according to the manufacturer's protocol.

The SCARA5 surface localization was assessed by Flow cytometry (FACS) and confocal microscopy analyses of HeLa transfected cells.

For Flow Cytometry analysis, HeLa cells transfected with each construct or with the empty vector (2x10⁴ per well) were pelletted in 96 U-bottom microplates by centrifugation at 200 x g for 5 min at 4°C and incubated for 1 hour at 4°C with the appropriate dilutions of anti-SCARA5-antibodies. The cells were washed twice in PBS-5% FCS and incubated for 20 min with the appropriate dilution of R-Phycoerythrin (PE)-conjugated secondary antibodies (Jackson Immuno Research, PA, USA) at 4°C. After washing, cells were analysed by a FACS Canto II flow cytometer (Becton Dickinson). Data were analyzed with FlowJo 8.3.3 program.

For confocal microscopy, transfected and control HeLa cells were plated on glass cover slips and after 48 h were washed with PBS and fixed with 3% formaldheyde solution in PBS for 20 min at RT. Then, after extensive washing in PBS, the cells were incubated with the antibodies overnight at 4°C (1:200) with or without a previous permeabilization step with 0.01% BriJ96® (Fluka). Cells were then stained with Alexafluor 488-labeled goat anti-mouse antibodies (Molecular Probes). DAPI (Molecular Probes) was used to visualize nuclei. The cells were mounted with glycerol plastine and observed under a laser-scanning confocal microscope (LeicaSP5).

### Results

The complete coding sequence for the target protein SCARA5 was cloned in the eukaryotic expression vector pcDNA3.1, sequence- verified and and used for transient transfection of HeLa cells. Expression of the target protein was detected by Western blot in total protein extracts from HeLa cells transfected with the SCARA5-encoding construct using the anti-SCARA5 antibody. Two bands of approximately 60 and 70KDa were visible in HeLa cells transfected with plasmid expressing the SCARA5 495 amino acid-protein isoformwhile the same bands was not visible in HeLa cells transfected with the empty pcDNA3 plasmid. The 60Kda protein species shows the expected molecular mass, while the 70KDa species likely corresponds to a SCARA5 form carrying post-translational modifications (e.g. glycolyslation). This confirmed that the antibody recognized specifically its target protein. A second band of approximately 39KDa was visible both in transfected and control HeLa cells, which could correspond to another SCARA5 isoform (known to exist), endogenously expressed by the cells. Results are represented in Figure 6A.

Surface localization of target proteins was addressed by FACS and confocal microscopy analyses of transiently transfected cells stained with the specific antibodies. Data are reported for cells transfected with the construct encoding the SCARA5 495 amino acid isoform. In this experiment the SCARA5- antibody was capable of binding the surface of transfected HeLa cells, while no binding was observed on cells transfected with the empty pcDNA3 vector (Figure 6B). A similar result was obtained when transfectd HeLa cells were analysed by confocal microscopy. As shown in Figure 6C, the anti-SCARA5 antibody was able to stain the plasma membrane of transfected cells expressing SCARA5, with or without cell permeabilization with the detergent. This indicates that this target protein is localized on the cell surface and is accessible to the external environment.

### Example 5. Expression and localization of SCARA5 protein in tumor cell lines

SCARA5 expression was also assessed by WB on total extracts from a panel of human epithelial cell lines representing the tumors analised by IHC. In each analysis, cells were cultured in under ATCC recommended conditions, and sub-confluent cell mono-layers were detached with PBS-0.5 mM EDTA and lysed by several freeze-thaw passages in PBS-1% Triton. Total protein extracts were loaded on SDS-PAGE (2x10⁵ cells/lane), and subjected to WB with specific antibodies as described above. (Figure 7A)

The marker cellular localization was assessed by Flow cytometry and confocal microscopy analyses on tumor cell lines, using the above described procedures (see Example 5).

### Results

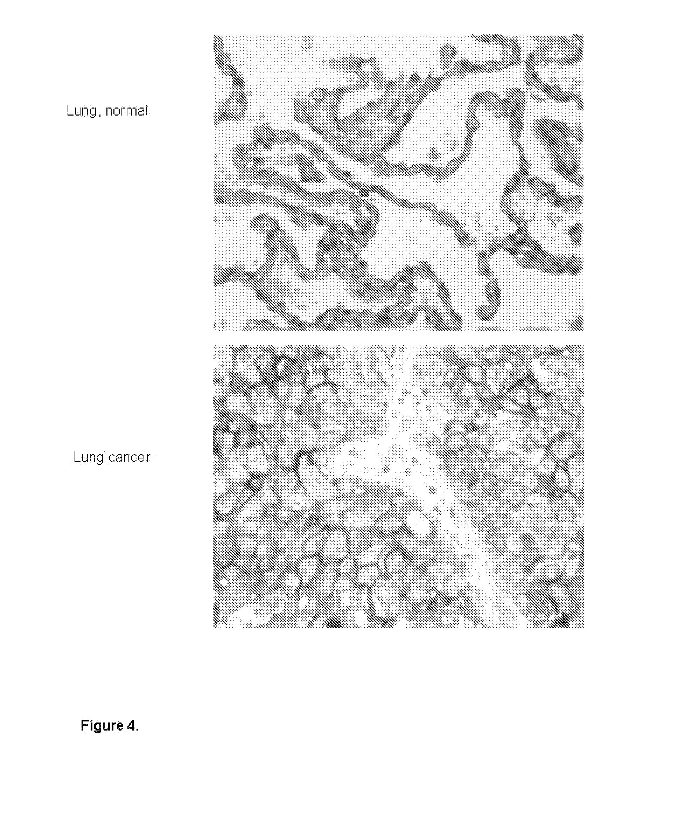

SCARA5 expression was confirmed in a panel of human tumor cell lines from breast, colon, lung and ovary tumors, examples of which are given in Figure 4.

In particular, SCARA5 expression is reported for a panel of the tumor cell lines including Ovcar3, Ovcar4, Ovcar5, Ovcar8 (ovary adenocarcinoma), and HCT-15 and Colo205 (colorectal cancer), H226 (lung tumor), MDA-MB231 and SKBr3 (breast cancer). In all tested cell lines different protein species were detected by the antibody, including a major protein band of approximately 40 KDa and other proteins species of approximately 20, 50 and 60 KDa, that could correspond to the annotated SCARA5 isoforms (Figures 7A). Surface staining of a panel of tumor cell lines indicates that SCARA5 protein is at least partially exposed on the surface of tested cells, as judged by the capability of the anti-SCARA5 antibody to bind the cell surface. An example of FACS analysis is given for the ovary tumor cell line OvCar8 (Figure 7B). FACS results were also confirmed by confocal microscopy analysis of the same tumor cell lines, among which OvCar8 is represented in Figure 7C. As shown in the figure, the anti-SCARA5 antibody was able to detect the protein on the cell surface (Figure 7C). Both confocal microscopy and FACS data show that the protein is accessible to the external environment and this evidence suggests that SCARA5 could be exploited as therapeutic target of anticancer therapies.

### Example 6. Detection of target protein in tumor tissue homogenates

The presence of protein bands corresponding to SCARA5 protein was also investigated in tissue homogenates of tumor biopsies as compared to normal tissues from patients. Homogenates were prepared by mechanic tissue disruption in buffer containing 40 mM TRIS-HCl, 1 mM TCEP {Tris(2-carboxyethyl)-phosphine hydrochloride, Pierce} and 6M guanidine hydrochloride, pH 8. Western blot was performed by separation of the total protein extracts (20 µg/lane) proteins were detected by specific antibodies. An example of this analysis is given for the detection of SCARA5 in lung tumor homogenates

### Results

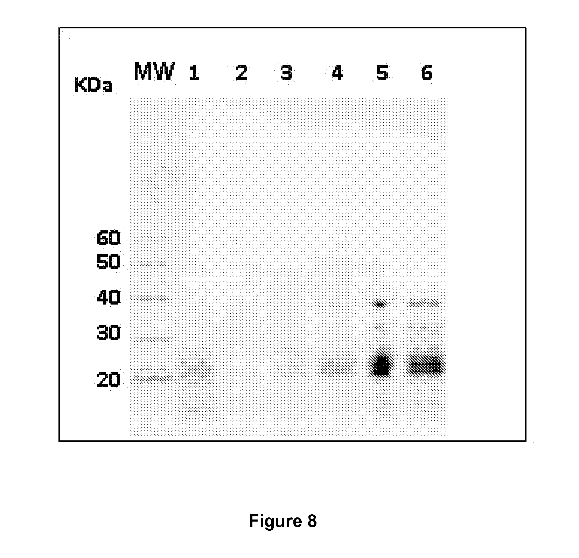

The anti-SCARA5 antibodies detected specific protein bands in homogenates from tumor samples (ranging from 40 to 20 KDa), that could correspond to the annotated SCARA5 isoforms confirming the presence of the marker proteins in lung tumor. Results are reported in Figure 8.

### References

1) Adams G.P. and Weiner L.M. (2005) Monoclonal antibody therapy cancer. Nat Biotechnol. 23:1147-1157.
2) Anderson, L., and Seilhamer, J. (1997). A comparison of selected mRNA and protein abundances in human liver. Electrophoresis 18: 533 - 537.
3) Chen, G., Gharib, T. G., Wang, H., Huang, C. C., Kuick, R., Thomas, D. G., Shedden, K. A., Misek, D. E., Taylor, J. M., Giordano, T. J., Kardia, S. L., Iannettoni, M. D., Yee, J., Hogg, P. J., Orringer, M. B., Hanash, S. M., and Beer, D. G. (2003) Protein profiles associated with survival in lung adenocarcinoma. Proc. Natl. Acad. Sci. U. S. A 100: 13537 - 13542.
4) Ginestier, C., Charafe-Jauffret, E., Bertucci, F., Eisinger, F., Geneix, J., Bechlian, D., Conte, N., Adelaide, J., Toiron, Y., Nguyen, C., Viens, P., Mozziconacci, M. J., Houlgatte, R., Birnbaum, D., and Jacquemier, J. (2002) Distinct and complementary information provided by use of tissue and DNA microarrays in the study of breast tumor markers. Am. J. Pathol. 161: 1223-1233.
5) Gygi, S. P., Rochon, Y., Franza, B. R., and Aebersold, R. (1999) Correlation between protein and mRNA abundance in yeast. Mol. Cell. Biol. 19, 1720 -1730; Nishizuka, S., Charboneau, L., Young, L., Major, S., Reinhold, W. C., Waltham, M., Kouros-Mehr, H., Bussey, K. J., Lee, J.K., Espina, V., Munson, P. J., Petricoin, E., III, Liotta, L. A., and Weinstein, J.N. (2003) Proteomic profiling of the NCI-60 cancer cell lines using new high-density reverse-phase lysate microarrays. Proc. Natl. Acad. Sci. U.S.A. 100, 14229 - 14234.
6) Tyers, M., and Mann, M. (2003) From genomics to proteomics. Nature 422: 193 - 197.
7) Li JY, Paragas N, Ned RM, Qiu A, Viltard M, Leete T, Drexler IR, Chen X, Sanna-Cherchi S, Mohammed F, Williams D, Lin CS, Schmidt-Ott KM, Andrews NC, Barasch J. Scara5 is a ferritin receptor mediating non-transferrin iron delivery. Dev Cell. (2009) 16:35-46.
8) Huang J, Zheng DL, Qin FS, Cheng N, Chen H, Wan BB, Wang YP, Xiao HS, Han ZG. Genetic and epigenetic silencing of SCARA5 may contribute to human hepatocellular carcinoma by activating FAK signaling. J Clin Invest. (2010) 120:223-41.
9) Aslanidis C, de Jong PJ. (1990) Ligation-independent cloning of PCR products (LIC-PCR). Nucleic Acids Res. 18:6069-74.
10)Studier FW. (2005) Protein production by auto-induction in high density shaking cultures. Protein Expr Purif. 41:207-34.
11) Kononen J, Bubendorf L, Kallioniemi A, Bärlund M, Schraml P, Leighton S, Torhorst J, Mihatsch MJ, Sauter G, Kallioniemi OP. Tissue microarrays for high-throughput molecular profiling of tumor specimens. (1998) Nat Med. 4:844-7.
12) Kallioniemi OP, Wagner U, Kononen J, Sauter G. (2001) Tissue microarray technology for high-throughput molecular profiling of cancer. Hum Mol Genet. 10:657-62.

## Claims

1. A tumor marker for use in the detection of breast, colon, lung and ovary cancer, wherein said tumor marker is:
(i) SCARA5 protein in one of its variant isoforms SEQ ID NO:1, SEQ ID NO:2, SEQ ID NO:3, SEQ ID NO:4 or a different isoform having sequence identity of at least 80%, preferably at least 90%, more preferably at least 95% to SEQ ID NO:1, SEQ ID NO:2, SEQ ID NO:3 or SEQ ID NO:4, or
(ii) a nucleic acid molecule containing a sequence coding for a SCARA5 protein, said encoding sequence being preferably SEQ ID NO:5, SEQ ID NO:6, SEQ ID NO:7 or SEQ ID NO:8.

2. A method of screening a sample of breast, colon, lung or ovary tissue for malignancy, said method comprising determining the presence in said sample of a tumor marker according to claim 1.

3. A method according to claim 2, wherein said tumor marker is a SCARA5 protein, said method being based on immunoradiometric, immunoenzymatic or immunohistochemical techniques.

4. A method according to claim 2, wherein said tumor marker is a SCARA5 nucleic acid molecule, said method being based on polymerase chain reaction techniques.

5. A method *in vitro* for determining the presence of a breast, colon, lung or ovary tumor in a subject, comprising the steps of:
(a) providing a sample of the tissue suspected of containing tumor cells;
(b) determining the presence of a tumor marker according to claim 1 in said tissue sample by detecting the expression of the marker protein or the presence of the respective mRNA transcript;
wherein the detection of one or more tumor markers in the tissue sample is indicative of the presence of tumor in said subject.

6. A method of screening compounds for antitumor candidates, which comprises contacting cells expressing a tumor marker protein according to claim 1 with the test compound and determining the binding of said compound to said cells.

7. An antibody or a fragment thereof which is able to specifically recognize and bind the tumor marker protein according to claim 1.

8. An antibody according to claim 7, which is either monoclonal or polyclonal.

9. The use of an antibody according to claim 7 or 8, for the preparation of a therapeutic agent for the treatment of proliferative diseases, preferably of breast, colon, lung and ovary cancer.

10. A diagnostic kit containing an antibody according to claims 7-8 and/or an oligonucleotide complementary to a nucleic acid molecule encoding a tumor marker according to claim 1, and optionally reagents, buffers, solutions and materials to carry out an immunoassay or a PCR assay.
